Europäisches Patentamt

European Patent Office

Office européen des brevets

(11) Veröffentlichungsnummer: **0 256 437 B1**

(12) **EUROPÄISCHE PATENTSCHRIFT**

(45) Veröffentlichungstag der Patentschrift: **04.09.91**

(51) Int. Cl.⁵: **A61N 1/365**

(21) Anmeldenummer: **87111352.8**

(22) Anmeldetag: **05.08.87**

(54) **Messvorrichtung zur Steuerung implantierbarer Körperersatzteile.**

(30) Priorität: **18.08.86 DE 3627933**

(43) Veröffentlichungstag der Anmeldung:
**24.02.88 Patentblatt 88/08**

(45) Bekanntmachung des Hinweises auf die
Patenterteilung:
**04.09.91 Patentblatt 91/36**

(84) Benannte Vertragsstaaten:
**DE FR GB IT NL SE**

(56) Entgegenhaltungen:
**EP-A- 0 096 464
EP-A- 0 133 828
WO-A-85/05279
DE-A- 1 573 240**

**F. Kohlrausch, "Praktische Physik", Band 2,
23. Auflage, Teubner Stuttgart 1985, Seite 59**

(73) Patentinhaber: **Siemens Aktiengesellschaft
Wittelsbacherplatz 2
W-8000 München 2(DE)**

(72) Erfinder: **Heinze, Roland, Dipl.-Ing.
Simbacher Strasse 9
W-8000 München 80(DE)**
Erfinder: **Liess, Hans-Dieter, Prof. Dr.
Pfaffenkamer Strasse 5
W-8193 Münsing(DE)**

## Beschreibung

Die Erfindung betrifft eine Meßvorrichtung zur Steuerung implantierbarer Körperersatzteile mit einer Pulssteuerschaltung, an die über einen Katheter mit Stromleitern eine Meßsonde angeschlossen ist.

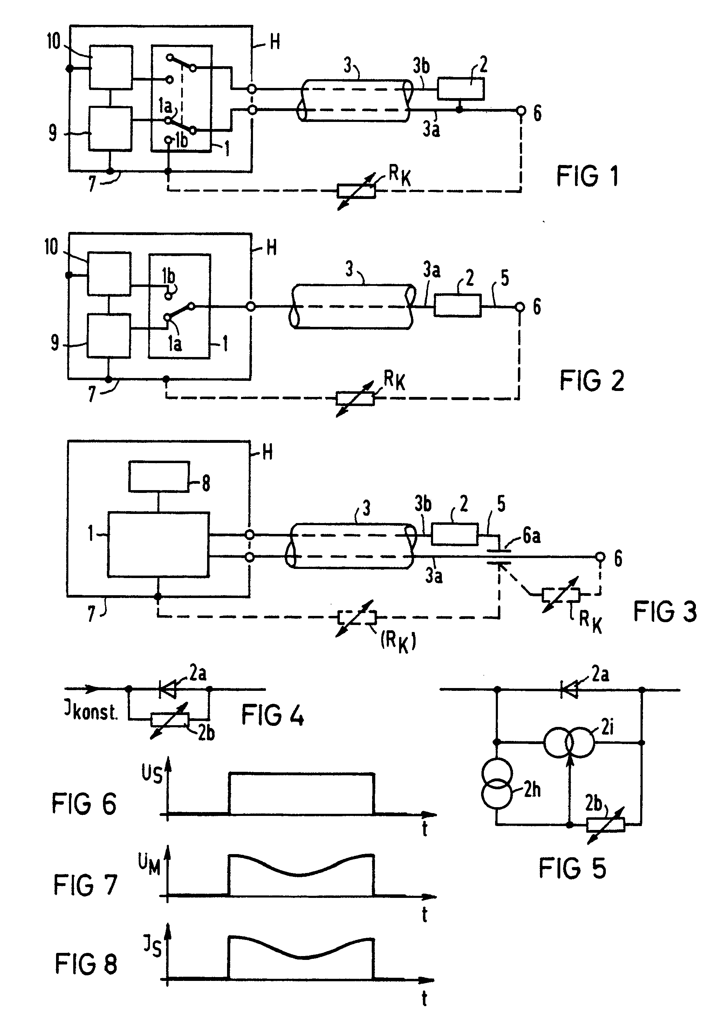

Eine derartige Meßvorrichtung zur Erfassung der Blutsauerstoffsättigung für die Frequenzregelung eines Herzschrittmachers ist beispielsweise aus der DE-C 31 52 963 bekannt. Der schematische Aufbau einer solchen bekannten Meßvorrichtung ist in Fig. 1 dargestellt.

Bei einem Herzschrittmachersystem der bekannten Art, bestehend aus dem Herzschrittmacher H und dem kombinierten Meß- und Stimulationskatheter 3 werden von einer Pulssteuerschaltung 1 der Katheter 3 abwechselnd mit einer Schrittmacherschaltung 9 und der sie steuernden Meßschaltung 10 verbunden. Mit der Pulssteuerschaltung 1 werden dabei zwei Betriebszustände des Systems eingestellt, wobei bei Schalterstellung 1a die Schrittmacherschaltung 9 über den Stromleiter 3a und die Elektroden 6 und 7 (Gehäuse des Herzschrittmachers H) sowohl das an den Elektroden anliegende EKG-Signal erfaßt als auch den Stimulationsimpuls abgibt. Bei Schalterstellung 1b der Pulssteuerschaltung 1 tastet die Meßschaltung 10 über die Stromleiter 3a und 3b die Meßsonde 2 ab.

Der Katheter muß daher zumindest zwischen Pulssteuerschaltung 1 des Herzschrittmachers H und Meßsonde 2 zweipolig ausgeführt werden. Zweipolige Katheter sind jedoch dicker und dementsprechend weniger flexibel und haben eine höhere Ausfallwahrscheinlichkeit als einpolige Katheter. Außerdem ist die Herstellung zweipoliger Meßkatheter aufwendiger und teurer als die einpoliger Katheter.

Aus dem Buch F. Kohlrausch "Praktische Physik", Band 2, 23. Auflage, Teubner Verlag Stuttgart, Seite 59 ist es bekannt, bei der Messung großer Widerstände diese über ein Strommeßgerät an eine Spannungsquelle anzuschließen und die von der Spannungsquelle abgegebene Spannung mit einem Spannungsmeßgerät zu erfassen. Aus den gemessenen Werten von Strom und Spannung kann dann der Widerstandswert ermittelt werden.

Aufgabe der Erfindung ist es, eine Meßvorrichtung der eingangs genannten Art so auszugestalten, daß der Katheter flexibler und zuverlässiger gestaltet werden kann und seine Herstellung kostengünstiger ist.

Diese Aufgabe wird erfindungsgemäß dadurch gelöst, daß der Katheter mit einem einzigen Stromleiter zwischen Pulssteuerschaltung und Meßsonde ausgeführt ist und daß über Körperelektroden das Körpergewebe als zweiter Stromleiter zwischen Meßsonde und Pulssteuerschaltung benutzt wird.

Damit entfällt im Katheter zwischen Pulssteuerschaltung und Meßsonde eine Stromleitung, so daß dieser flexibler, zuverlässiger und kostengünstiger wird.

Vorteilhafterweise kann ein Meßsensor den Widerstand der Meßsonde so steuern, daß Änderungen des Stromflusses durch den gesamten Meßkreis abhängig vom Meßwert, aber unabhänig von Widerstandsänderungen des Körpergewebes sind. Der Meßkreis beinhaltet dabei Meßsonde und Stromleitungen.

Bei einer vorteilhaft einfachen Ausgestaltung kann der Meßsensor ein im Meßkreis liegender Widerstand sein, dessen meßgrößenabhängige Widerstandsänderung sehr groß gegenüber den als Störgrößen wirkenden Widerstandsänderungen der Stromleiter ist, so daß die Änderungen des Widerstandswertes des Körpergewebes in einem bestimmten Meßbereich vernachlässigbar klein bleiben.

Eine weitere Möglichkeit, das Meßergebnis vom Widerstand des Körpergewebes unabhängig zu machen, besteht darin, daß der Meßsensor einen seriell im Meßkreis liegenden Konstantstromregler steuert.

Das Problem, den Einfluß von Widerstandsänderungen des Körpergewebes auszuschalten, kann auch dadurch gelöst werden, daß die Meßsonde auf einen von der Pulssteuerschaltung ausgesandten Meßimpuls mit einer Widerstandsänderung reagiert, die gegenüber dem Meßimpuls eine meßwertabhängige Verzögerung $\Delta t$ aufweist. Diese pulsförmige Widerstandsänderung ist gegenüber den langsamen Änderungen des Widerstands des Körpergewebes eindeutig erfaßbar.

In einer weiteren alternativen Ausführungsform setzt die Meßsonde den Meßwert in eine digital codierte Impulsfolge um, entsprechend der sich der Widerstand der Meßsonde ändert. Diese digitale Information bleibt ebenfalls von langsamen Änderungen des Widerstands im Meßkreis unbeeinflußt.

Wird anstelle der Analog-Digitalwandlerschaltung ein Analog-Frequenzwandler verwendet, ergibt sich eine weitere Ausführungsform, bei der der Widerstand der Meßsonde frequenzmoduliert wird und der Meßwert als Frequenzinformation ebenso unabhängig von Widerstandsänderungen des Körpergewebes übertragen werden kann.

Bei einer Meßvorrichtung für einen Herzschrittmacher, bei dem die Pulssteuerschaltung zusätzlich Stimulationsimpulse über einen Katheter mit einer Stimulationselektrode an den Herzmuskel abgibt, kann die Meßsonde vorteilhafterweise seriell in die Verbindungsleitung zwischen Pulssteuerschaltung und Elektrode geschaltet sein, wobei die Meßsonde eine Diode enthält, die für die Stimulationsimpulse in Durchlaßrichtung und für die Meßimpulse in Sperrichtung liegt. Damit werden die Stimulationsimpulse nicht durch den meßgrößenabhängigen Widerstand der Meßsonde geschwächt.

Bei einer Meßvorrichtung, bei der die Pulssteuerschaltung einen Stimulationsimpuls über einen Kondensator abgibt, der sich in der Stimulationsphase über den Körperwiderstand entlädt und der anschließend in der Depolarisationsphase wieder aufgeladen wird, kann der zur Wiederaufladung nötige Strom, der Teil der Verlustenergie des Herzschrittmachers ist, vorteilhafterweise zur Ansteuerung der Meßsonde genutzt werden, indem die Pulssteuerschaltung den in umgekehrter Stromrichtung zum Stimulationsimpuls abgegebenen Meßimpuls ebenfalls über den Kondensator steuert.

Ausführungsbeispiele der Erfindung werden nachfolgend anhand der Figuren 2 bis 19 näher erläutert.

Figur 2 zeigt das Prinzip der Anordnung für einen Herzschrittmacher H. Die Pulssteuerschaltung 1, die den Katheter 3 abwechselnd an die Schrittmacherschaltung 9 oder die Meßschaltung 10 schaltet, ist über einen einadrigen Stromleiter 3a, der in dem Katheter 3 angeordnet ist, mit einem Anschluß einer in den Katheter 3 eingebauten Meßsonde 2 verbunden. Der zweite Anschluß der Meßsonde 2 ist über einen zweiten Abschnitt 5 des Stromleiters, der ebenfalls in dem Katheter 3 angeordnet ist, mit einer Stimulationselektrode 6 verbunden. Als Rückleiter zum Herzschrittmacher H dient das Körpergewebe, das durch den variablen Körperwiderstand $R_K$ gekennzeichnet ist, und die Gehäuseelektrode 7. Damit werden sowohl Stimulationsimpulse als auch Meßwerte über den Stromleiter 3a und 5 sowie das Körpergewebe (gestrichelte Linie) übertragen. Der Katheter 3 muß durchgehend nur mit einem Stromleiter 3a ausgeführt werden, so daß seine Flexibilität und Zuverlässigkeit steigt.

Figur 3 zeigt eine weitere Ausführungsform für eine Meßvorrichtung mit dem Körpergewebe als Rückleitung für die Meßsonde 2. In diesem Falle weist der Katheter 3 in bekannter Weise zusätzlich zur Stimulationselektrode 6 eine sogenannte indifferente Elektrode 6a auf. Solche indifferenten Elektroden werden benötigt, wenn die Stimulation und EKG-Messung mit dem Herzschrittmacher H möglichst störungsfrei und deshalb nicht über den Körperkreis vorgenommen werden soll, sondern im Herzen selbst zwischen Stimulationselektrode 6 und indifferenter Elektrode 6a durchgeführt wird. In diesem Falle erfolgt der Stromfluß durch die Meßsonde 2 über die Leitungsabschnitte 3b und 5 zur indifferenten Elektrode 6a und von da über das venöse Blut und den Herzmuskel $R_H$ zur Stimulationselektrode 6 zurück über die Leitung 3a zur Pulssteuerschaltung 1. In diesem Falle weist zwar der Katheter 3 auch einen zweiten Leiter 3b auf, als Vorteil dieser Anordnung bleibt jedoch, daß die Meßsonde 2 lediglich zwei Anschlüsse aufweist. Beim Stand der Technik gemäß Figur 1 sind dagegen drei Anschlüsse erforderlich, nämlich zwei Anschlüsse zum Herzschrittmacher H und ein weiterführender Anschluß zur Stimulationselektrode 6. In der Sondenherstellung sind jedoch Anschlüsse kompliziert und die Ausfallwahrscheinlichkeit wird durch jeden Anschluß statistisch erhöht.

Figur 4 zeigt schematisch eine Schaltungsvariante für die Meßsonde 2. Diese enthält die Parallelschaltung einer Diode 2a und eines Meßsensors, der in diesem Falle als meßgrößenabhängiger Widerstand 2b dargestellt ist. Für eine Temperaturmessung kann der Widerstand 2b z.B. ein temperaturabhängiger Widerstand sein. Die Diode 2a ist so gepolt, daß sie für den Stimulationsimpuls leitend ist. Damit wird verhindert, daß der Stimulationsimpuls durch einen Spannungsabfall am Widerstand 2b geschwächt wird.

Die Messung wird durch eine Widerstandsmessung mit einem Konstantstrom durchgeführt, der in Sperrichtung der Diode 2a liegt.

Bei dieser einfachen Ausführungsform ist vor allem der Störeinfluß durch schwankenden Körperwiderstand $R_K$ sowie die bei jeder Stimulation an der Elektrode 6 entstehende Polarisationsspannung störend. Der Einfluß dieser Störgrößen kann jedoch dadurch vernachlässigbar klein gemacht werden, daß die meßgrößenabhängige Widerstandsänderung des Widerstands 2b groß gegenüber den als Störgrößen wirkenden Widerstandsänderungen der Verbindungsleitungen einschließlich Körperwiderstand $R_K$ ist.

Eine weitere Möglichkeit zur Eliminierung des Störeinflusses des schwankenden Körperwiderstandes $R_K$ ist in Figur 5 dargestellt. Dabei ist in der Meßsonde 2 der Diode 2a eine steuerbare Stromquelle 2i parallel geschaltet. Eine zweite Stromquelle 2h versorgt den meßgrößenabhängigen Widerstand 2b. Ein Steuereingang der Stromquelle 2i ist mit dem Verbindungspunkt von Stromquelle 2h und meßgrößenabhängigem Widerstand 2b verbunden.

Bei Beaufschlagung der Schaltung mit einem Meßimpuls $U_S$ fließt durch die Stromquelle 2h ein Konstantstrom und erzeugt an dem meßwertabhängigen Widerstand 2b eine meßwertabhängige Steuerspannung $U_M$, die die steuerbare Stromquelle 2i steuert. In Figur 6 ist der an der Meßsonde 2 anstehende Meßimpuls $U_S$ dargestellt. Figur 7 zeigt die an der steuerbaren Stromquelle 2i anstehende meßwertabhängige Steuerspannung $U_M$. Die steuerbare Stromquelle 2i liefert aufgrund dieser Steuerspannung $U_M$ einen der Steuerspannung $U_M$ und damit dem Meßwert proportionalen Konstantstrom $I_S$, der in der Meßschaltung 10 gemessen werden kann. Dieser Konstantstrom $I_S$ ist in einem vorgegeben Bereich unabhängig vom Widerstand der Verbindungsleitungen einschließlich Körperwiderstand $R_K$ und von den Polarisationsspannung an den Elektroden 6,7.

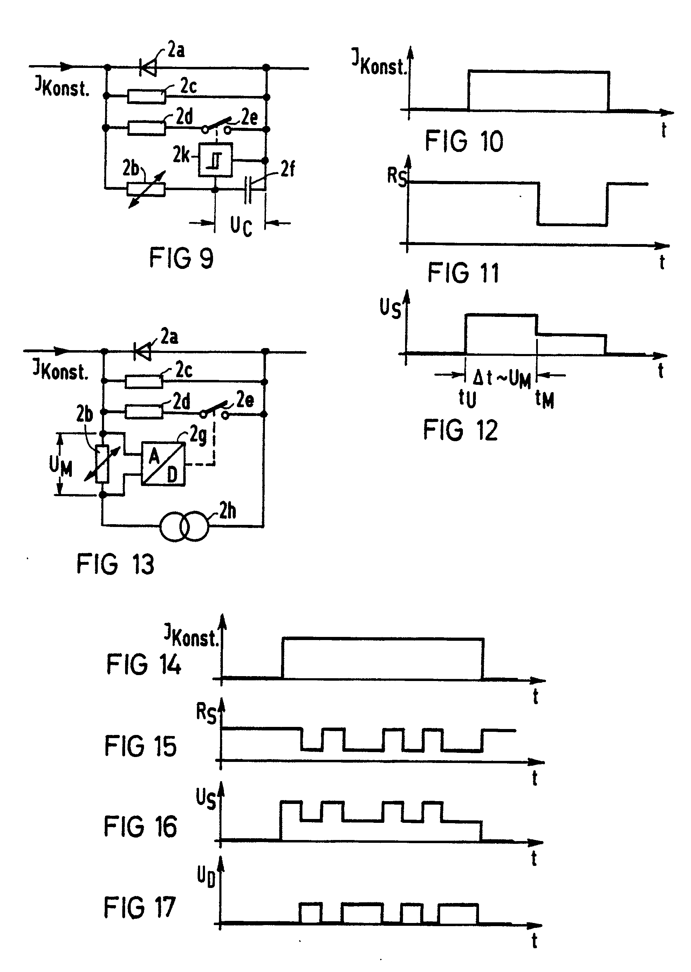

Eine weitere Möglichkeit, die Einflüsse von sich änderndem Körperwiderstand $R_K$ und Polarisationsspannungen zu eliminieren, besteht darin, daß die Meßsonde auf einen von der Auswerteschaltung ausgesandten Meßimpuls $U_S$ mit einer Widerstandsänderung reagiert, die gegenüber dem Meßimpuls $U_S$ eine meßwertabhängige Verzögerung aufweist. Ein Schaltungsbeispiel zur Realisierung dieser Meßwerterfassung ist in Figur 9 dargestellt. Dabei ist der Diode 2a ein Widerstand 2c, die Reihenschaltung eines Widerstandes 2d und eines Schalters 2e und die Reihenschaltung des meßgrößenabhängigen Widerstands 2b und eines Kondensators 2f parallel geschaltet. Der Schalter 2e wird über einen Schwellwertschalter 2k von der am Kondensator 2f anstehenden Spannung $U_c$ gesteuert.

Die Funktion der Schaltung wird nachfolgend anhand der Diagramme nach den Figuren 10-12 erläutert. Wenn man die Schaltung mit einem Konstantstrom-Meßimpuls ($I_{Konst}$) entgegengesetzt zur Leitrichtung der Diode 2a beaufschlagt, so ist der Schalter 2e zunächst noch geöffnet und der gesamte wirksame Sondenwiderstand $R_S$ der Meßsonde 2 wird im wesentlichen durch den Widerstand 2c bestimmt.

Damit liegt an der Meßsonde 2 der Meßimpuls $U_S$. Gleichzeitig wird der Kondensator 2f über den meßgrößenabhängigen Widerstand 2b aufgeladen. Nach einer Zeit $\Delta t$, die vom Widerstandswert des meßgrößenabhängigen Widerstands 2b und damit von der Meßgröße abhängig ist, erreicht die Spannung $U_c$ am Kondensator 2f den Schwellwert des Schalters 2k, der den Schalter 2e schließt. Damit wird nun auch der Widerstand 2d wirksam, so daß der Sondenwiderstand $R_S$ und damit auch die Spannung des Meßimpulses $U_S$ abfällt. Die Zeitspanne $\Delta t$ zwischen Beginn $t_U$ des Meßimpulses $I_{Konst}$ und dem Zeitpunkt $t_M$ des Spannungsabfalls des Meßimpulses $U_S$ stellt somit ein Maß für die Meßgröße dar, das problemlos unabhängig von den langsamen Änderungen des Widerstandes $R_K$ des Körpergewebes und von Polarisationsspannungen erfaßt werden kann.

Schließlich gibt es auch die Möglichkeit, die Meßgröße unabhängig von Widerstandsschwankungen des Körpergewebes und von Polarisationsspannungen digital zu übertragen. Figur 13 zeigt schematisch ein Ausführungsbeispiel für eine solche Anordnung. Dabei ist der Diode 2a ein Widerstand 2c, die Reihenschaltung eines Widerstands 2d und eines Schalters 2e sowie die Reihenschaltung des meßgrößenabhängigen Widerstands 2b und einer Konstantstromquelle 2h parallel geschaltet. Ein Analog-Digitalwandler 2g greift eingangsseitig die meßwertabhängige Steuerspannung $U_M$ vom meßgrößenabhängigen Widerstand 2b ab und steuert ausgangsseitig den Schalter 2e.

Wenn man diese Schaltung mit einem Meßimpuls $I_{Konst}$ entgegengesetzt zur Leitrichtung der Diode 2a beaufschlagt, so steht am Eingang des Analog-Digital-Wandlers 2g eine meßwertabhängige Steuerspannung $U_M$ an. Diese Spannung wird nun in einen Digitalwert codiert, der den Schalter 2e steuert und damit entsprechend den Widerstand 2d zuschaltet. Die digitale Information wird daher zur Auswerteschaltung aufgrund der in Figur 15 dargestellten Änderungen des Sondenwiderstands $R_S$ bzw. der Sondenspannung $U_S$ übertragen. Dabei kann der Analog-Digital-Wandler 2g so ausgeführt sein, daß er nach jedem Meßimpuls $I_{Konst}$ zunächst einen Startimpuls abgibt und dann den erfaßten Meßwert z.B., wie in den Figuren 14-16 dargestellt, in Form einer Binär-Information überträgt. Figur 17 zeigt die daraus gebildete digitale Meßspannung $U_D$.

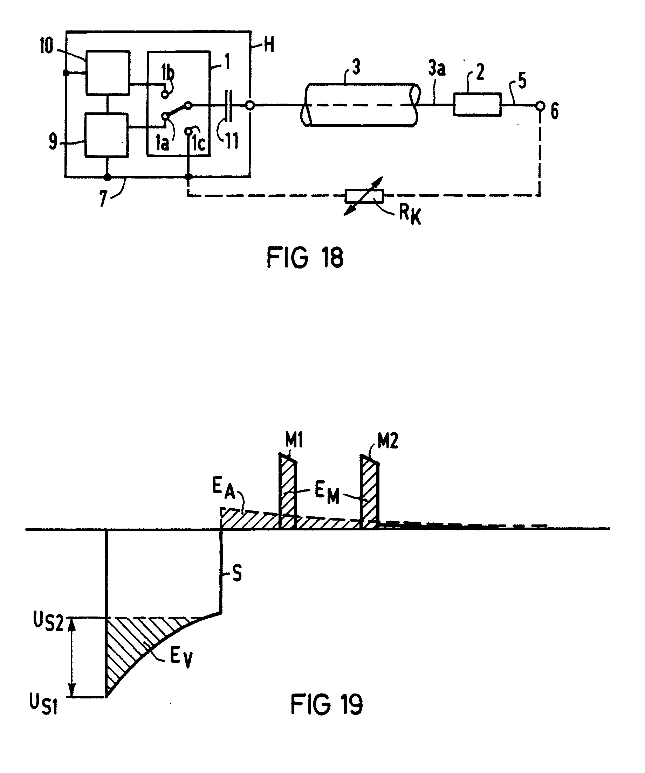

Figur 18 zeigt ein Ausführungsbeispiel, bei dem der Herzschrittmacher H einen Kondensator 11 enthält, über den der Stimulationsimpuls geleitet wird.

Mit dem erfindungsgemäßen einpoligen Meßkatheter 3 ist es auf einfache Weise möglich, die Energie, die beim Wiederaufladen eines Kondensators 11, der in dem Stimulationskreis der Schrittmacherschaltung 9 genutzt wird, sonst verloren geht, für die Messung zu nutzen.

Gegenüber dem Ausführungsbeispiel nach Figur 2 ist zusätzlich der Kondensator 11 zwischen die Pulssteuerschaltung 1 und den Stromleiter 3a geschaltet und die Pulssteuerschaltung 1 weist eine dritte Schalterstellung 1c auf, um nach der Meßphase die Restladung des Kondensators 11 an Masse abzuleiten.

Figur 19 verdeutlicht anhand des katheterseitigen Spannungsverlaufs am Kondensator 11 die Wirkung der Meßimpulssteuerung. Dabei ist mit S der über den Kondensator 11 abgegebene Stimulationsimpuls bezeichnet. Die im Spannungsabfall des Stimulationsimpulses S von $U_{S1}$ nach $U_{S2}$ enthaltene Verlustleistung Ev durch Entladung des Kondensators 11 über den Körperwiderstand $R_K$ wird normalerweise in der Wiederaufladephase (gestrichelte Linie) ausgeglichen. Die dazu nötige Aufladeleistung $E_A$ kann durch entsprechende Meßimpulssteuerung zum Beispiel für zwei Meßimpulse M1,M2 mit dem Leistungsbedarf $E_M$ genutzt werden.

**Patentansprüche**

1. Meßvorrichtung zur Steuerung implantierbarer Körperersatzteile mit einer Pulssteuerschaltung (1), an die über einen Katheter (3) mit Stromleitern (3a,b) eine Meßsonde (2) angeschlossen ist,

**dadurch gekennzeichnet,**

daß der Katheter (3) mit einem einzigen Stromleiter (3a) zwischen Pulssteuerschaltung (1) und Meßsonde (2) ausgeführt ist und daß über Körperelektroden (6,7) das Körpergewebe als zweiter Stromleiter zwischen Meßsonde (2) und Pulssteuerschaltung (1) benutzt wird.

2. Meßvorrichtung nach Anspruch 1, **dadurch gekennzeichnet, daß** ein Meßsensor (2b) den Widerstand der Meßsonde (2) so steuert, daß Änderungen des Stromflusses durch den gesamten Meßkreis abhängig vom Meßwert, aber unabhängig vom Widerstand ($R_K$) des Körpergewebes sind.

3. Meßvorrichtung nach Anspruch 1 oder 2, **dadurch gekennzeichnet,** daß ein von der Pulssteuerschaltung (1) abgegebener Meßimpuls so kurz und energiearm ist, daß keinerlei Eigenaktivität des Körpergewebes ausgelöst wird.

4. Meßvorrichtung nach einem der Ansprüche 1 bis 3, **dadurch gekennzeichnet,** daß der Stromkreis über das Körpergewebe nach dem Meßimpuls oder nach den Meßimpulsen solange geschlossen bleibt, bis sich die nach der Meßphase an den Elektroden (6,7) vorhandenen Polarisationsspannungen abgebaut haben.

5. Meßvorrichtung nach einem der Ansprüche 1 bis 4, **dadurch gekennzeichnet,** daß der Meßsensor (2b) ein im Meßkreis liegender Widerstand ist, dessen meßgrößenabhängige Widerstandsänderung sehr groß gegenüber den als Störgrößen wirkenden Widerstandsänderungen der Verbindungsleitungen ist.

6. Meßvorrichtung nach einem der Ansprüche 1 bis 4, **dadurch gekennzeichnet,** daß der Meßsensor (2b) einen seriell im Meßkreis liegenden Konstantstromregler (2i) steuert.

7. Meßvorrichtung nach einem der Ansprüche 1 bis 4, **dadurch gekennzeichnet,** daß die Meßsonde (2) auf einen von der Pulssteuerschaltung (1) ausgesandten Meßimpuls ($I_{Konst}$) mit einer Widerstandsänderung reagiert, die gegenüber dem Meßimpuls ($I_{Konst}$) eine meßwertabhängige Verzögerung aufweist.

8. Meßvorrichtung nach einem der Ansprüche 1 bis 4, **dadurch gekennzeichnet,** daß die Meßsonde (2) den Meßwert in eine digital codierte Impulsfolge umsetzt, entsprechend der sich der Widerstand der Meßsonde (2) ändert.

9. Meßvorrichtung nach einem Anspruch 1, **dadurch gekennzeichnet,** daß die Meßsonde (2) den Meßwert in eine Frequenz umsetzt, entsprechend der sich der Widerstand der Meßsonde (2) ändert.

10. Meßvorrichtung nach einem der Ansprüche 1 bis 9 für einen Herzschrittmacher (H), wobei die Pulssteuerschaltung (1) zusätzlich Stimulationsimpulse über den Katheter (3) mit Stromleiter (3a) und Stimulationselektrode (6) an den Herzmuskel ($R_H$) abgibt, **dadurch gekennzeichnet,** daß die Meßsonde (2) in die Verbindungsleitung (3a,5) zwischen Pulssteuerschaltung (1) und Elektrode (6) seriell geschaltet ist und daß die Meßsonde (2) eine Diode (2a) enthält, die für die Stimulationsimpulse in Durchlaßrichtung und für die Meßimpulse in Sperrichtung liegt.

11. Meßvorrichtung nach Anspruch 10, wobei die Pulssteuerschaltung (1) einen Stimulationsimpuls über einen Kondensator ($C_S$) abgibt, der sich in der Stimulationsphase über den Körperwiderstand ($R_K$) entlädt und der anschließend in der Depolarisationsphase wieder aufgeladen wird, **dadurch gekennzeichnet,** daß der zur Wiederaufladung nötige Strom, der Teil der Verlustenergie des Herzschrittmachers (H) ist, zur Ansteuerung der Meßsonde (2) genutzt wird, indem die Pulssteuerschaltung (1) den in umgekehrter Stromrichtung zum Stimulationsimpuls abgegebenen Meßimpuls ebenfalls über den Kondensator ($C_S$) führt.

## Claims

1. A measuring device far controlling implantable body replacement parts with a pulse control circuit (1), to which there is connected a measuring probe (2), by way of a catheter (3) with current conductors (3a, b), characterised in that the catheter (3) between pulse control circuit (1) and measuring probe (2) is constructed with a single current conductor (3a) and in that by way of body electrodes (6,7) the body tissue is used as second current conductor between the measuring probe (2) and the pulse control circuit (1).

2. A measuring device according to claim 1, characterised in that a measuring sensor (2b) controls the resistance of the measuring probe (2), such that alterations of the flow of current through the whole measuring circuit are dependent on the measured value, but independent of the resistance ($R_K$) of the body tissue.

3. A measuring device according to claim 1 or 2, characterised in that a measuring pulse delivered by the pulse control circuit (1) is short and low in energy so that no internal activity of the body tissue is triggered.

4. A measuring device according to one of claims 1 to 3, characterised in that the current circuit through the body tissue remains closed after the measuring pulse or measuring pulses, until the polarization voltages present at the electrodes (6,7) after the measuring phase have reduced.

5. A measuring device according to one of claims 1 to 4, characterised in that the measuring sensor (2b) is a resistor lying in the measuring circuit, the measurement-size-dependent resistance change of which is very large relative to the resistance changes of the connecting lines acting as interference.

6. A measuring device according to one of claims 1 to 4, characterised in that the measuring sensor (2b) controls a constant current regulator (2i) lying in series in the measuring circuit.

7. A measuring device according to one of claims 1 to 4, characterised in that the measuring probe (2) reacts to a measuring pulse ($I_{const}$) emitted by the pulse control circuit with a resistance change, which has a measurement-value-dependent delay, relative to the measuring pulse ($I_{const}$).

8. A measuring device according to one of claims 1 to 4, characterised in that the measuring probe (2) transforms the measured value into a digitally coded pulse series, corresponding to which the resistance of the measuring probe (2) changes.

9. A measuring device according to claim 1, characterised in that the measuring probe (2) transforms the measured value into a frequency, corresponding to which the resistance of the measuring probe (2) changes.

10. A measuring device according to one of claims 1 to 9, for a heart pacemaker (H), wherein the pulse control circuit (1) delivers, in addition, stimulation pulses by way of the catheter (3) with the current conductor (3a) and stimulation electrode (6), to the heart muscles ($R_H$), characterised in that the measuring probe (2) is connected in series in the connector (3a, 5) between pulse control circuit (1) and electrode (6), and in that the measuring probe (2) contains a diode (2a), which lies in the conducting direction for the stimulation pulses and in the non-conducting direction for the measuring pulses.

11. A measuring device according to claim 10, wherein the pulse control circuit (1) delivers a stimulation pulse by way of a capacitor ($C_S$), which discharges in the stimulation phase through the body resistance ($R_K$) and which is charged again subsequently in the depolarization phase, characterised in that the current which is necessary for recharging, which is part of the energy loss of the heart pace maker (H), is used to control the measuring probe (2), as the pulse control circuit (1) also feeds the measuring pulse delivered in the reverse current direction to the stimulation pulse via the capacitor ($C_S$).

**Revendications**

1. Dispositif de mesure pour la commande de substituts implantables de parties du corps, comportant un circuit de commande impulsionnelle (1), auquel une sonde de mesure (2) est raccordée par l'intermédiaire d'un cathéter (3), au moyen de conducteurs de courant (3a,b), caractérisé par le fait que le cathéter (3) comporte un seul conducteur de courant (3a) entre le circuit de commande impulsionnel (1) et la sonde de mesure (2) et que le tissu corporel est utilisé comme second conducteur de courant entre la sonde de mesure (2) et le circuit de commande impulsionnelle (1), moyennant l'utilisation d'électrodes (6,7) placées sur le corps.

2. Dispositif de mesure suivant la revendication 1, caractérisé par le fait qu'un capteur de mesure (2b) commande la résistance de la chambre de mesure (2) de manière que des variations du flux de courant dans l'ensemble du circuit de mesure dépendent de la valeur de mesure, mais soient indépendantes de la résistance (R$_K$) du tissu corporel.

3. Dispositif de mesure suivant la revendication 1 ou 2, caractérisé par le fait qu'une impulsion de mesure délivrée par le circuit de commande impulsionnelle (1) est suffisamment brève et présente une énergie suffisamment faible pour ne déclencher aucune sorte d'activité propre du tissu corporel.

4. Dispositif de mesure suivant l'une des revendications 1 à 3, caractérisé par le fait que le circuit incluant le tissu corporel reste fermé après l'impulsion de mesure ou les impulsions de mesure jusqu'à ce que les tensions de polarisation présentes, après la phase de mesure, au niveau des électrodes (6,7) se soient évanouies.

5. Dispositif de mesure suivant l'une des revendications 1 à 4, caractérisé par le capteur de mesure (2b) est une résistance placée dans le circuit de mesure et dont la variation, qui dépend de la grandeur de mesure, est très importante par rapport aux variations de la résistance des conducteurs de liaison, qui agissent en tant que grandeurs parasites.

6. Dispositif de mesure suivant l'une des revendications 1 à 4, caractérisé par le fait que le capteur de mesure (2b) commande un régulateur de courant constant (2i) monté en série dans le circuit de mesure.

7. Dispositif de mesure suivant l'une des revendications 1 à 4, caractérisé par le fait que la sonde de mesure (2) réagit à une impulsion de mesure (I$_{constante}$) émise par le circuit de commande impulsionnelle (1), par une variation de la résistance, qui présente un retard dépendant de la valeur de mesure, par rapport à l'impulsion de mesure (I$_{constante}$).

8. Dispositif de mesure suivant l'une des revendications 1 à 4, caractérisé par le fait que la sonde de mesure (2) convertit la valeur de mesure en une suite d'impulsions codée numériquement, en fonction de laquelle varie la résistance de la sonde de mesure (2).

9. Dispositif de mesure suivant la revendication 1, caractérisé par le fait que la sonde de mesure (2) convertit la valeur de mesure en une fréquence, en fonction de laquelle varie la résistance de la sonde de mesure (2).

10. Dispositif de mesure suivant l'une des revendications 1 à 9 pour un stimulateur cardiaque (H), et dans lequel le circuit de commande impulsionnelle (1) envoie en outre, au muscle cardiaque (R$_H$), des impulsions de stimulation par l'intermédiaire du cathéter (3) comportant un conducteur de courant (3a) et une électrode de stimulation (6), caractérisé par le fait que la sonde de mesure (2) est branchée en série dans la ligne de jonction (3a,5) entre le circuit de commande impulsionnelle (1) et l'électrode (6), et que la sonde de mesure (2) contient une diode (2a), qui est passante pour les impulsions de stimulation et est bloquée pour les impulsions de mesure.

11. Dispositif de mesure suivant la revendication 10, dans lequel le circuit de commande impulsionnelle (1) envoie l'impulsion de stimulation par l'intermédiaire d'un condensateur (C$_S$), qui se décharge pendant la phase de stimulation par l'intermédiaire de la résistance (R$_K$) du corps et est ensuite à nouveau chargé pendant la phase de dépolarisation, caractérisé par le fait que le courant, qui est nécessaire pour la charge et fait partie de l'énergie dissipée du stimulateur cardiaque (H), est utilisé pour la commande de la sonde de mesure (2), par le fait que le circuit de commande impulsionnelle (1) envoie également par l'intermédiaire du condensateur (C$_S$), l'impulsion de mesure délivrée dans le sens de courant, inverse de celui de l'impulsion de stimulation.

FIG 1

FIG 2

FIG 3

FIG 4

FIG 5

FIG 6

FIG 7

FIG 8

FIG 9

FIG 10

FIG 11

FIG 12

FIG 13

FIG 14

FIG 15

FIG 16

FIG 17

FIG 18

FIG 19